(19) Europäisches Patentamt
European Patent Office
Office européen des brevets

(11) Publication number: 0 339 815 A2

(12) # EUROPEAN PATENT APPLICATION

(21) Application number: 89303419.9

(22) Date of filing: 06.04.89

(51) Int. Cl.4: C07C 61/37 , C07C 61/35 , C07C 51/00 , C07C 49/323 , C07C 143/54

The title of the invention has been amended (Guidelines for Examination in the EPO, A-III, 7.3).

(30) Priority: 23.04.88 GB 8809652

(43) Date of publication of application: 02.11.89 Bulletin 89/44

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Krief, Alain
34 Rue Tienne aux Clochers
B-5150 Wepion(BE)

(74) Representative: Bishop, Nigel Douglas et al
Imperial Chemical Industries PLC Legal Department: Patents PO Box 6 Bessemer Road
Welwyn Garden City Herts, AL7 1HD(GB)

(54) **Process for the preparation of pure cis-cyclopropane carboxylic acids and intermediates therefor.**

(57) A process for the preparation of cis-cyclopropane carboxylic acids which comprises the step of treating a derivative of a 4-hydroxy-2,2-dimethyl bicyclo[3,1,0]hexan-2-one with a source of hydroxide ions.

EP 0 339 815 A2

## CHEMICAL PROCESS & INTERMEDIATES

This invention relates to a novel process for obtaining substantially pure cis-cyclopropane carboxylic acids such as cis-chrysanthemic acid without the parallel production of trans-cyclopropane carboxylic acids, and to novel intermediates useful in the process.

The process enables production of cis-chrysanthemic acid, a valuable intermediate from which known insecticidal products can be obtained by esterification with, for example, allethrolone or 5-benzyl-3-furylmethanol, by a route which avoids the concommitant production of the trans isomer.

The process of the invention comprises as its principal step the ring-opening of a compound of formula (I) :

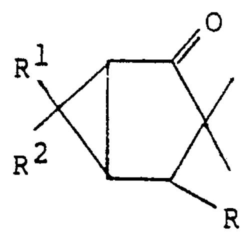

by treatment with a source of hydroxide ions for example an alkali metal hydroxide. In the compounds of formula (I) R can be any atom or group which is capable of leaving by forming an anion under the reaction conditions and may include, for example, a halogen atom, an alkoxy group, or, preferably, a esterified hydroxy group, such as the methane sulphonyloxy group, and $R^1$ and $R^2$ are each selected from hydrogen and methyl.

This step of the process is preferably carried out in an aqueous medium in the presence of an aprotic solvent such as dimethylsulphoxide.

The compound of formula (I) where $R^1$ and $R^2$ are both methyl and R is an esterified hydroxy group may be obtained by esterification of 4-hydroxy-3,3,6,6-tetramethylbicyclo[3,1,0]hexan-2-one by reaction with an acid halide or anhydride. Thus 3,3,6,6-tetramethylbicyclo[3,1,0]hexa-2-on-4-yl methane sulphonate and 3,4,6,6-tetramethylbicyclo[3,1,0]hexan -2-on-4-yl 4-methylbenzene sulphonate may be obtained by reacting the 4-hydroxy compound with methane sulphonyl chloride, and 4-methylbenzene sulphonyl chloride respectively

The 4-hydroxy compound may be obtained by selective reduction of the known compound 3,3,6,6-tetramethylbicyclo[3,1,0]hexa-2,4-dione, using for example an alkali metal borohydride such as lithium, sodium or potassium borohydride. This reduction gives rise to a mixture of exo- and endo-4-hydroxy compounds. However, the ratio or exo to endo isomers may vary with the choice of reducing agent used. Thus, for example, a product consisting of a major amount of the endo-isomer and a minor amount of the exo-isomer can be obtained by reduction of this dione with sodium borohydride alone, whereas the use of sodium borohydride in the presence of a cerium salt such as cerium chloride yields only the exo-isomer. It appears that the exo-isomer is preferentially esterfied when a mixture with the endo-isomer is reacted with methane sulphonyl chloride or 4-methylbenzene sulphonyl chloride.

Although the 3,3,6,6-tetramethylbicyclo[3,1,0]hexa-2,4-dione is a known material, the present invention includes, as one aspect thereof, an improved process for the preparation of the dione which gives the product in high yield without the co-production of by-products from which it cannot be readily separated. The process comprises the steps of (a) treating 2,2,5,5-tetramethyl-hexa-1,3-dione with a least one equivalent of base in an aprotic medium, (b) thereafter treating the resultant mixture with bromine, and (c) recovering the product from the mixture. A suitable aprotic medium is tetrahydrofuran, and a suitable base is an alkali metal alkoxide, such as potassium t-butoxide.

Similar processes to those described above may be applied to the compounds of formula I where $R^1$ and $R^2$ are both hydrogen or one of $R^1$ and $R^2$ is hydrogen and the other is methyl, to give the cis-isomers of 2-(2-methylpropen-1-yl)cyclopropane carboxylic acid and 2-methyl-3-(2-methylpropen-1-yl)cyclopropane carboxylic acid.

Further details of these processes may be ascertained from the Examples hereinafter.

In a further aspect the invention provides novel compounds useful as intermediates in these processes, of formula:-

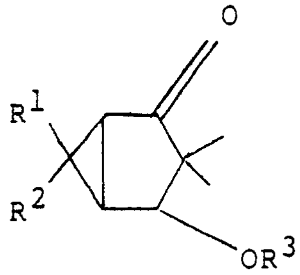

(II)

where $R^1$ and $R^2$ are each selected from hydrogen and methyl and $R^3$ is hydrogen, or a substituted sulphonyl group.

Specific compounds of formula II useful as intermediates include the endo and exo isomeric forms of the following:

4-hydroxy-3,3,6,6-tetramethylbicyclo[3,1,0]hexan-2-one
4-hydroxy-3,3,6-trimethylbicyclo[3,1,0]hexan-2
4-hydroxy-3,3-dimethylbicyclo[3,1,0]hexan-2-one
3,3,6,6-tetramethylbicyclo[3,1,0]hexan-2-on-4-yl methane sulphonate
3,3,6,6-tetramethylbicyclo[3,1,0]hexan-2-on-4-yl 4-methylbenzene sulphonate
3,3,6-trimethylbicyclo[3,1,0]hexan-2-on-4-yl methane sulphonate
3,3-dimethylbicyclo[3,1,0]hexan-2-on-4-yl methane sulphonate

The cyclopropane acids obtained by process of the invention are exclusively in the form in which the 2-methylpropenyl group and the carboxylic acid group are in a cis-relationship. The compounds cis-2-methyl-3-(2-methylpropen-1-yl)cyclopropane carboxylic acid and cis-2-(2-methylpropen-1-yl) cyclopropane carboxylic acid appear not to have been described previously.

The sequence of process steps involved in these processes is set out in the following Scheme:

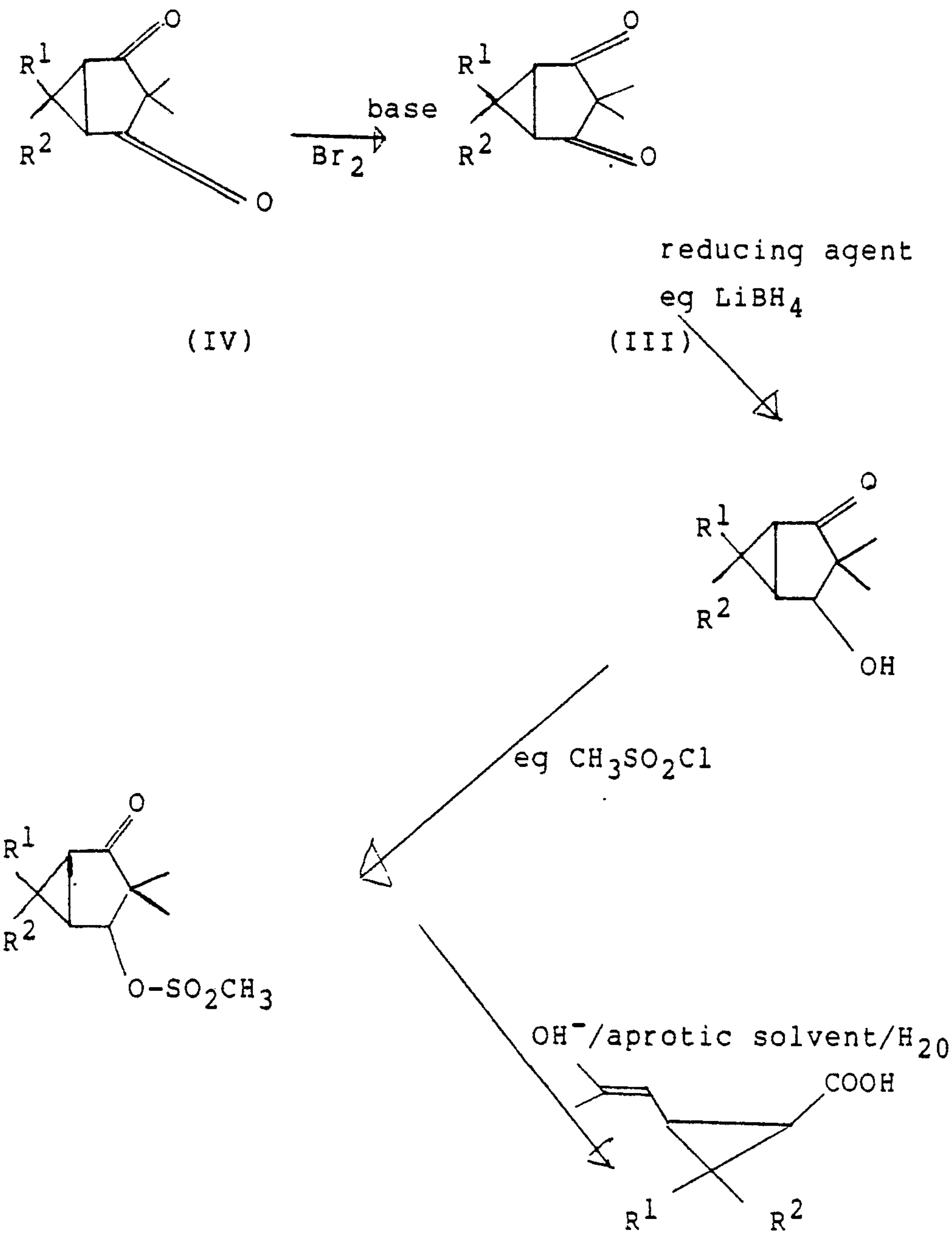


(IV)

(III)

The compounds of formula III and formula IV where $R^1$ is hydrogen and $R^2$ is methyl appear not to have been described previously.

It will be appreciated by those skilled in the art that the bicyclic diones of formula III may be subjected to reduction to the hydroxy ketones in an asymmetric manner to yield compounds predominantly of one optically active isomeric form by the use of chiral reducing systems, in particular those using enzymes or microbes, such as for example those described by Mori, H et al (Tetrahedron, 1988, 44 343 and Agric. Biol. Chem 1985, 49 2373) Brooks, D,w et al (J. Org. Chem, 1982, 47 2820) or Djerassi, C et al (J. Org. Chem, 1983 48 4549).

The various aspects of the invention are illustrated but not limited, by reference to the following Examples.

## EXAMPLE 1

This Example illustrates the preparation of 3,3,6,6-tetramethylbicyclo[3,1,0]hexa-2,4-dione.

A solution of potassium t-butoxide (24.7g) in anhydrous tetrahydrofuran (150 $cm^3$) was added dropwise under an argon atmosphere to a solution of 2,2,5,5-tetramethylcyclohexa-1,3-dione (16.1 g) in anhydrous tetrahydrofuran (150 $cm^3$) maintained at -78°C. A solution of bromine (26.9g) in pentane (30 $cm^3$) is then added slowly whilst the solution is maintained at 40°C, after which the resultant mixture is stirred for a further 1 hour. The reaction is quenched with aqueous phosphate buffer solution (50 $cm^3$, pH7) and the aqueous phase washed with diethyl ether (2 x 5 $cm^3$). The ether washings were combined with the organic phase and the whole dried over anhydrous magnesium sulphate. After filtering and removal of the organic solvents by evaporation under reduced pressure the residue (23.2g) was purified by column chromatography on silica gel by elution with an ether-pentane mixture (20:80 by volume) to yield 3,3,6,6-tetramethylbicyclo[3,1,0]hexa-2,4-dione (10.7g) b.p. 75°C/5 mm Hg, Rf 0.33 (TLC $SiO_2$/benzeneethylacetate, 96:4).

| | |
|---|---|
| $^1$H NMR ($CCl_4$) ppm : | 1.0 (s, 3H); 1.2 (s, 6H); 1.3 (s, 3H); 2.3 (s, 2H). |
| Infra red (neat) : | 2962, 2924, 1725, 1608, 1532, 1380, 1260, 1041, 857 $cm^{-1}$. |
| Mass Spectrum : | 168 ($M^+$), 153, 140, 108. |
| Microanalysis : | calculated C, 72.3; H, 8.5%<br>found C, 72.1; H, 8.3% |

## EXAMPLE 2

This Example illustrates the preparation of 4-hydroxy-3,3,6,6-tetramethylbicyclo[3,1,0]hexan-2-one.

Lithium borohydride (0.5 $cm^3$ of a 2M solution in tetrahydrofuran) is added to stirred solution of 3,3,6,6-tetramethylbicyclo[3,1,0]hexa-2,4-dione (166 mg) in anhydrous diethyl ether (5 $cm^3$) maintained at 20°C under an argon atmosphere. After stirring the mixture for 30 minutes, water (1 $cm^3$) was added and the phases separated. The aqueous was extracted with diethyl ether (2 x 3 $cm^3$) and combined with the organic phase. After drying over anhydrous magnesium sulphate and filtering, the solvents were removed by evaporation under reduced pressure to yield the desired product (158 mg) as a mixture of the exo and endo stereoisomers (55:45), Rf 0.26 (TLC $SiO_2$/etherpentane, 1:1).

(a) Exo-4-hydroxy-3,3,6,6-tetramethylbicyclo[3,1,0]hexan-2-one

$^1$H NMR ($CDCl_3$) ppm : 1.00 (s, 3H); 1.05 (s, 3H); 1.10 (s, 3H); 1.15 (s, 3H); 1.72 (dd, 1H, J=5.5 Hz, J=1.0 Hz); 1.90 (dd, 1H, J=5.5 Hz, J=1.0 Hz); 3.10 (s, 1H); 3.91 (s, 1H).

Infra red (neat) : 3403, 2962, 2872, 1694, 1467, 1378, 110 $cm^{-1}$.

Mass Spectrum : 168 ($M^+$), 153, 140, 108.

(b) Endo-4-hydroxy-3,3,6,6-tetramethylbicyclo[3,1,0]-hexan-2-one

| | |
|---|---|
| $^1H$ NMR ($CDCl_3$) ppm: | 1.08 (s, 3H); 1.17 (s, 3H); 1.27 3H); 1.93 (d, 1H, J = 5.3 Hz); 2.10 (dd, 1H,J = 7 0 Hz, J = 5.3 Hz); 3.10 (s, 1H); (d, 1H, J = 7.0 Hz). |
| Infra red (neat) : | 3457, 2958, 2926, 1704, 1377, 1115, 1087 $cm^{-1}$. |
| Mass Spectrum : | 168 ($M^+$), 153, 140, 108. |

EXAMPLE 3

This Example illustrates the preparation of 3,3,6,6-tetramethylbicyclo[3,1,0]hexan-2-on-4-yl methane sulphonate.

Methane sulphonyl chloride (378 mg) is added at 0°C under an argon atmosphere to a solution of 4-hydroxy-3,3,6,6-tetramethylbicyclo[3,1,0]hexan-2-one (504 mg, 55:45 mixture of exo and endo stereoisomers) and triethyl-amine (0.7 $cm^3$) in dichloromethane (15 $cm^3$). After stirring the mixture for 1 hour at 0°C, cold water (3 $cm^3$) is added, the aqueous phase separated and extracted with dichloromethane (2 x 3 $cm^3$) and the extracts combined with the organic phase. After drying the organic phase over anhydrous magnesium sulphate and filtering the solvents were removed by evaporation under reduced pressure to yield a viscous oil (635 mg) comprising unreacted endo-4-hydroxy-3,3,6,6-tetramethylbicyclo[3,1,0]hexan-2-one (221 mg) and 3,3,6,6-tetramethylbicyclo[3,1,0]hexan-2-on-4-yl methane sulphonate, Rf 0.68 (TLC $SiO_2$/benzene-ethyl acetate, 80:20). The desired product was isolated by the mixture by preparative layer chromatography ($SiO_2$/benzene-ethylacetate 70:30).

$^1H$ NMR ($CDCl_3$) ppm : 4.6 (s, 1H); 4.1 (s, 3H); 2.0 (s, 2H); 1.1 (M).

Infra red (neat) : 2971, 2937, 1726, 1465, 1176, 949 $cm^{-1}$

EXAMPLE 4

This Example illustrates the preparation of cis-chrysanthemic acid.

A mixture of 3,3,6,6-tetramethylbicyclo[3,1,0]hexan-2-on-4-yl methane sulphonate (120 mg), potassium hydroxide (700 mg), water (0.4 $cm^3$) and dimethylsulphoxide (1.6 $cm^3$) was heated at 70°C for 4 hours, cooled to 20°C and diluted with water (4 $cm^3$) and diethyl ether (4 $cm^3$). The aqueous phase was separated, washed with diethyl ether (4 $cm^3$), acidified with 2N hydrochloric acid (3 $cm^3$) and extracted with diethyl ether (2 x 5 $cm^3$). The ethereal extracts were combined with the organic phase from the reaction, dried over anhydrous magnesium sulphate, filtered, and concentrated by evaporation of the solvent under reduced pressure to yield cis-chrysanthemic acid (38 mg), identified by comparison with an authentic sample.

EXAMPLE 5

This Example illustrates the preparation of 3,3,6,6-tetramethylbicyclo[3,1,0]hexan-2-on-4-yl 4-methylbenzene sulphonate.

4-methylbenzene sulphonyl chloride (228mg) is added at 0 °C under an argon atmosphere to a mixture of 4-hydroxy-3,3,6,6-tetramethylbicyclo[3,1,0]hexan-2-one (168mg, 55:45 mixture of exo and endo stereoisomers), pyridine ($0.32cm^3$), dimethylaminopyridine (24mg) and dichloromethane ($3.0cm^3$). After the addition was completed, the resultant solution was warmed to the ambient temperature (ca.22 °C) and stirred for 16 hours. Water cooled to 0 °C ($1.0cm^3$) was then added and the aqueous phase separated, extracted twice with dichloromethane ($2.0cm^3$) and the extracts combined with the organic phase. After drying the organic phase over anhydrous sodium sulphate and filtering, the solvents were removed by evaporation under reduced pressure to yield a viscous oil (320mg). comprising unreacted endo-4-hydroxy-3,3,6,6-tetramethylbicyclo[3,1,0]hexan-2-one (68mg) and 3,3,6,6-tetramethylbicyclo[3,1,0]hexan-2-on-4-yl 4-methylbenzene sulphonate, (158mg) rf 0.54 (TLC $SiO_2$) diethyl ether-pentane,1:1). The desired product was separated from the mixture by preparative layer chromatography ($SiO_2$) diethyl ether-pentane, 1:1), as a white solid, m.p. 106 °C.

| | |
|---|---|
| $^1$H NMR ($CDCl_3$) ppm: | 7.60 (dd,2H); 4.50 (s,1H); 2.50 (s,3H); 1.76 (dd,2H); 1.08 (s,6H); 1.00 (s,3H); 0.92 (s,3H). |
| Infra red (paraffin mull): | 2929, 1726, 1363, 1190 $cm^{-1}$. |

EXAMPLE 6

This Example illustrates the preparation of cis-chrysanthemic acid.

A mixture of 3,3,6,6-tetramethylbicyclo[3,1,0]hexan-2-on-4-yl 4-methylbenzene sulphonate (161mg), potassium hydroxide (168mg), dimethylsulphoxide ($4.5cm^3$) and water ($0.5cm^3$) is heated at 70 °C for 2.5 hours, cooled to 20 °C, and diluted with water ($6.0cm^3$) and diethyl ether ($4.0cm^3$). The aqueous phase was separated, washed with diethyl ether ($2.0cm^3$), acidified with 2N hydrochloric acid ($3.0cm^3$) and extracted with diethyl ether (2 x $5.0cm^3$). The ethereal extracts were combined with the organic phase from the reaction, dried over anhydrous magnesium sulphate, filtered, and concentrated by evaporation of the solvent under reduced pressure to yield cis-chrysanthemic acid (67mg), indentified by comparison with an authentic sample.

| | |
|---|---|
| $^1$H NMR ($CDCl_3$) ppm: | 5.30 (d,2H); 1.66 (m,6H), 1.3 (m,6H). |
| Infra red (neat): | 2963, 2930, 1697, 1434, 1377, 1225 $cm^{-1}$. |

EXAMPLE 7

This Example illustrates the preparation of 2,2-dimethylcyclohexan-1,3-dione.

Methyl iodide (57.3 $cm^3$) was added to a mixture of cyclohexan-1,3-dione (40g), potassium carbonate (100g), ethanol (150 $cm^3$) and water (150 $cm^3$) at this ambient temperature (ca. 22 °C), and the resultant mixture heated at 70 °C for 4 hours, and then cooled at 20 °C. The mixture was then extracted with diethyl ether (4 x $400cm^3$). The combined extracts were dried over anhydrous magnesum sulphate, filtered, and concentrated by evaporation of the solvents under reduced pressure to give a residual oil (37g) which was purified by column chromatography (silica gel eluted with a diethyl ether - pentane mixture, 1:1) to yield 2,2-dimethylcyclohexan-1,3-dione (32g), Rf 0.8 (TLC,$SiO_2$, diethyl ether-pentane, 1:1).

| | |
|---|---|
| $^1H$ NMR ($CDCl_3$) ppm: | 1.3 (s,6H); 2.0 (m.2H); 2.7 (m,4H). |
| Infra red (neat): | 2991, 2966, 1726, 1696, 1459, 1029 $cm^{-1}$ |
| Mass spectrum: | 168 ($M^+$), 97, 70. |
| Microanalysis: | calculated C, 68.5; H, 8.61% |
| | found C, 68.47; H, 8.82% |

EXAMPLE 8

This Example illustrates the preparation of 3,3-dimethylbicyclo[3,1,0]hexa-2,4-dione.

A solution of potassium t-butoxide (7.4g) in anhydrous tetrahydrofuran ($60cm^3$) was added dropwise to a stirred solution of 2,2-dimethylcyclohexa-1,3-dione (4.24g) in anhydrous tetrahydrofuran ($60cm^3$) maintained at -78°C. A gummy yellowish precipitate was formed, after which the temperature of the mixture was raised to 40°C and a solution of bromine (7.6g) in pentane ($5.0cm^3$) was added slowly. After stirring for a further hour at this temperature, the mixture was quenched with an aqueous phosphate buffer solution (50 $cm^3$, pH7), the aqueous phase separated, extracted with diethyl ether ($2x10cm^3$) and the extracts combined with the organic phase from the reaction, and dried over anhydrous magnesium sulphate. After filtration this solvents were removed by evaporation under reduced pressure and the residue (4.97g) was subjected to column chromatographic purification (silica gel eluted with diethyl ether-pentane mixture,1:1) to yield 3,3-dimethylbicyclo[3,1,0]hexa-2,4-dione (2.8g, TLC Rf 0.37, $SiO_2$ ether-pentane,1:1)

| | |
|---|---|
| $^1H$ NMR ($CDCl_3$) ppm: | 1.0 (s,6H); 1.5 (m,2H); 2.5 (q,2H). |
| Infra red (neat): | 2968, 1726, 1608, 1686, 1029 $cm^{-1}$ |
| Mass spectrum: | 138 ($M^+$), 123, 110. |
| Microanalysis: | calculated C, 69.5; H, 7.3 % |
| | found C, 69.38; H, 7.31 % |

EXAMPLE 9

This Example illustrates the preparation of 4-hydroxy-3,3-dimethylbicyclo[3,1,0]hexane-2-one.

Lithium borohydride in tetrahydrofuran ($0.5cm^3$ of a 2M solution) is added dropwise to a stirred solution of 3,3-dimethylbicyclo [3,1,0]hexa-2,4-dione (138mg) in anhydrous diethyl ether ($5.0cm^3$) under an argon atmosphere at the ambient temperature (ca. 22°C), the mixture stirred for 30 minutes, and thereafter water ($1.0cm^3$) was added. The aqueous phase was extracted with diethyl ether (2 x $3cm^3$) and the extracts combined with the organic phase from the reaction, and dried over anhydrous magnesium sulphate. Evaporation of the solvents under reduced pressure gave a residual viscous oil (120mg) which comprised 4-hydroxy-3,3-dimethylbicyclo[3,1,0] hexane-2-one as a mixture of the exo and endo stereoisomers (15:85), Rf 0.26 (TLC $SiO_2$/ether-pentane, 1:1).

| | |
|---|---|
| $^1H$ NMR ($CDCl_3$) ppm: | 1.00 (s,3H); 1.10 (m,3H); 1.30 (m,2H); 2.30 (m,2H); 4.08 (s,1H); 4.33 (d,1H). |
| Infra red (neat): | 3417, 2974, 1707, 1466, 1314, 1066, 961, 869 $cm^{-1}$. |

EXAMPLE 10

This Example illustrates the preparation of 3,3-dimethylbicyclo[3,1,0]hexan-2-on-4-yl methane sulphonate. Methane sulphonyl chloride (7mg) and triethylamine ($0.2cm^3$) are added at 0°C to a solution of 4-hydroxy-3,3-dimethylbicyclo[3,1,0]hexan-2-one (189mg, exo/endo 8:92) in dichloromethane ($5.0cm^3$) and the

reaction mixture is stirred for 1 hour, after which water (0° C, $3cm^2$) is added. The aqueous phase is separated, extracted with dichloromethane ($2x3cm_3$) and the extracts combined with the organic phase from the reaction and dried over anhydrous magnesium sulphate. After filtration and evaporation of the solvents under reduced pressure, the residual oil (320mg) is subjected to layer chromatographic purification ($SiO_2$/ benzene-ethyl acetate, 70:30) to yield 3,3-dimethylbicyclo[3,1,0]hexane-2-on-4-yl methane sulphonate (244mg), Rf 0.45 (TLC $SiO_2$/benzene-ethyl acetate, 70:30).

| | |
|---|---|
| $^1H$ NMR ($CDCl_3$) ppm: | 1.00 (s,3H); 1.15 (s,3H); 1.30 (m,2H); 2.30 (m,2H); 3.08 (s,3H); 5.08 (d,1H). |
| Infra red (neat): | 1354, 1177, 947, 923, 793 $cm^{-1}$ |
| Mass spectrum: | 190, 122, 111, 79. |
| Microanalysis: | calculated C, 49.5; H, 6.5 %<br>found C, 49.33; H, 6.41 % |

## EXAMPLE 11

This Example illustrates the preparation of cis-2-(2-methylpropen-1-yl) cyclopropane carboxylic acid.

A mixture of 3,3-dimethylbicyclo[3,1,0]hexan-2-on-4-yl methane sulphonate (260mg), potassium hydroxide (403mg), dimethylsulphoxide ($1.6cm^3$) and water ($0.4cm^3$) is heated at 70° C for 1.5 hours, cooled to 20° C, and partitioned between water ($4.0cm^3$) and diethyl ether ($4.0cm^3$). The aqueous phase is separated, washed with diethyl ether ($4.0cm^3$), acidified with dilute (2N) hydrochloric acid ($3.0cm^3$) to pH2, and extracted with diethyl ether (2 x $5.0cm^3$). After drying the combined extracts over anhydrous magnesium sulphate and filtering, the solvents are removed by evaporation under reduced pressure to yield cis-2-(2-methylpropen-1-yl) cyclopropane carboxylic acid (143mg).

| | |
|---|---|
| $^1H$ NMR ($CDCl_3$) ppm: | 1.1 (m,2H); 1.6 (s,6H); 2.0 (m,2H); 5.0 (d,1H); 10.5 (s,1H). |
| Infra red (neat): | 2929, 2693, 2561, 1685, 1443, 1237, 916 $cm^{-1}$. |
| Mass spectrum: | 140 ($M^+$), 125, 97. |
| Microanalysis: | calculated C, 68.56; H, 8.61 %<br>found C, 68.54; H, 8.6 % |

## EXAMPLE 12

The procedure of Example 7 is used to prepare 2,2,5-trimethylcyclohexa-1,3-dione from 5-methylcyclohexa-1,3-dione, as follows:

Methyl iodide ($0.6cm^3$) is added to a mixture of 5-methylcyclohexa-1,3-dione (544mg), potassium carbonate (1.106g), ethanol (7.0 $cm^3$) and water ($7.0cm^3$) and the resultant mixture heated at 70° C for 4 hours. After cooling to 20° C the mixture is extracted with diethyl ether (4 x 20 $cm^3$) and the combined extracts dried over anhydrous magnesium sulphate. After filtration the solution was concentrated by evaporation of the solvent under reduced pressure and the residual oil (702mg) purified by chromatography using a silica gel column eluted with a 1:1 mixture of diethylether and pentane) to yield 2,2,5-trimethylcyclohexa-1,3-dione (382mg). Rf 0.75 (TLC $SiO_2$, ether-pentane, 1:1).

| | |
|---|---|
| $^1H$ NMR ($CDCl_3$)ppm: | 1.0 (d,3H); 1.15 (s,3H); 1.18 (s,3H); 2.5 (m,4H). |
| Infra red (neat): | 2991, 2966, 1726, 1696, 1459, 1029 $cm^{-1}$. |

## EXAMPLE 13

The procedure of Example 1 is used to prepare 3,3,6-trimethylbicyclo[3,1,0]hexa-2,4-dione from 2,2,5-trimethylcyclohexa-1,3-dione, as follows:
A solution of potassium t-butoxide (200mg) in dry tetrahydrofuran ($3.0cm^3$) is added dropwise under an argon atmosphere to a solution of 2,2,5-trimethylcyclohexa-1,3,dione (203 mg) in anhydrous tetrahydrofuran at -78° C. After the formation of a gummy precispitate is observed, bromine (312mg) in pantane ($1.0cm^3$) is slowly added to the mixture at 40° C, and the resultant mixture stirred for 1 hour. Aqueous phosphate buffer solution ($2.5cm^3$, pH7) is then added and the aqueous phase separated and extracted with diethyl ether (2 x $5.0cm^3$). The extracts are combined with the organic phase from the reaction, dried over anhydrous magnesium sulphate, filtered and concentrated by evaporation of the solvents under reduced pressure to give a residual oil (270mg). This is purified by column chromatography using a silica gel column eluted with a 1:1 mixture of diethyl ether and pentane, to yield 3,3,6-trimethylbicyclo[3,1,0]hexa-2,4-dione (130mg), Rf 0.5 (TLC, $SiO_2$, ether-pentane, 1:1).

| | |
|---|---|
| $^1H$, NMR ($CDCl_3$)ppm: | 1.0 (d,3H); 1.15 (s,3H); 1.2 (s,3H); 1.6 (m,1H); 2.15 (d,2H). |
| Infra red (neat): | 2968, 1726, 1608, 1686, 1029 $cm^{-1}$ |

EXAMPLE 14

The procedure of Example 2 (modified as described below) is used to prepare 4-hydroxy-3,3,6-trimethylbicyclo[3,1,0]hexa-2-one from 3,3,6-trimethylbicyclo[3,1,0]hexa-2,4-dione as follows:
Sodium borohydride (0.6mmole) is added at -78° C to a solution of 2,2,5-trimethylbicyclo [3,1,0]hexa-2,4-dione (90mg) in a methanol solution of cerium trichloride heptahydrate ($1.5cm^3$, 0.4M) and the mixture stirred for 5 minutes, after which water ($1.0cm^3$ is added). After separation the aqueous phase is extracted with diethyl ether (2 x $3.0cm^3$) and the extracts combined with the organic phase from this reaction and dried over anhydrous magnesium sulphate. After filtration the solvents are removed by evaporation under reduced pressure to yield a 47:53 mixture of the exo and endo isomers of 4-hydroxy-3,3,6-trimethylbicyclo [3,1,0]hexa-4-one (85mg), Rf 0.2 (TLC,$SiO_2$, ether-pentane, 1:1).

| | |
|---|---|
| $^1H$ NMR ($CDCl_3$)ppm: | 1.00 (m,9H); 1.75 (m,2H); 2.00 (m,1H); 2.66 (m,1H); 4.10 (s,1H); 4,25 (d,1H). |
| Infra red (neat): | 3514, 2965, 1707, 1461, 1063, 882 $cm^{-1}$ |

EXAMPLE 15

The procedure of Example 3 is used to prepare 3,3,6-trimethylbicyclo[3,1,0]hexan-2-on-4-yl methane sulphonate from 4-hydroxy-3,3,6-trimethylbicyclo[3,1,0]hexan-2-one as follows:
Methane sulphonyl chloride (32mg) and triethylamine (56mg) are added to a solution of 4-hydroxy-3,3,6-trimethylbicyclo[3,1,0]hexan-2-on-4-yl methane sulphonate (1.89mg, exo/endo ratio 47:53) in dichloromethane ($1.0cm^3$) at 0° C, and the resultant mixture stirred for 1 hour before cold water ($0.5cm^3$, 0° C) is added. After separation the aqueous phase is extracted with dichloromethane (2 x $3.0cm^3$) and the extracts combined with the organic phase from the reaction and dried over anhydrous magnesium sulphate. After filtration and evaporation of the solvent under reduced pressure the residual oil (158mg) was subjected to purification by column chromatogrpahy (silica gel column eluted with an 80:20 mixture of benzene and ethyl acetate) to yield 3,3,6-trimethylbicyclo[3,1,0]hexan-2-on-4-yl methane sulphonate (108mg), Rf0.35 (TLC, $SiO_2$, benzene-ethylacetate, 80:20).

| | |
|---|---|
| $^1H$ NMR ($CDCl_3$) ppm: | 1.17 (m,9H); 1.40 (m,1H); 2.00 (m,2H); 3.13 (s,3H); 3.16 (s,3H); 4.90 (s,1H); 5.08 (d,1H) |
| Infra red (neat): | 2973, 1727, 1351, 1174, 940, 793 $cm^{-1}$ |

EXAMPLE 16

The procedure of Example 4 is used to prepare cis-2-methyl-3-(2-methylpropen-1-yl)cyclopropropane carboxylic acid from 3,3,6-trimethylbicyclo[3,1,0]hexan-2-on-4-yl methane sulphonate as follows:
A solution of 3,3,6-trimethylbicyclo[3,1,0]hexan-2-on-4-yl methane sulphonate (46mg), and potassium hydroxide (67mg) in a mixture of dimethylsulphoxide ($1.0cm^3$) and water ($0.2cm^3$) is heated at 70°C for 15 minutes, and then cooled to 20°C and partioned between water ($3.0cm^3$) and diethyl ether ($5.0cm^3$). The aqueous phase is separated, washed with ether ($2.0cm^3$), acidified with dilute aqueous hydrochloric acid ($1.0cm^3$, 2N) to pH2, and extracted with diethyl ether (2 x $5.0cm^3$). The ethereal solution is dried over anhydrous magnesium sulphate, filtered and concentrated by evaporation of the solvent under reduced pressure to yield cis-2-methyl-3-(2-methylpropen-1-yl) cyclopropane carboxylic acid (22mg).

| | |
|---|---|
| $^1H$ NMR ($CDCl_3$)ppm: | 1.10 (m, 1H); 1.41 (d,3H,J=1.8 Hz); 1.72 (s,6H); 1.81 (m,2H); 5.10 (d,1H, J=1.0Hz). |
| Infra red (neat): | 2924, 2693, 1695, 1438, 1237 $cm^{-1}$ |
| Mass spectrum: | 154 ($M^+$), 139, 111. |

EXAMPLE 17

This Example illustrates the preparation of 4-hydroxybicyclo[3,1,0]hexan-2-ones by reduction of the corresponding 2,4-diones with various reducing agents.

Method A: Reduction with sodium borohydride.

This method is similar to that described in Example 2 and 9 except that an equivalent amount of sodium borohydride is used in place of lithium borohydride.

Method B: Reduction with sodium borohydride in the presence of cerium chloride [Luche, J-L, et al, J Amer. Chem. Soc., 103, 5454, (1981)]

The dione (1 mmole) is dissolved in a solution of cerium chloride heptahydrate in methanol ($2.5cm^3$, 0.4M), the solution cooled to -78°C and sodium borohydride (1 mmole) added. After reduction has occurred (the reaction may be monitored by TLC) the resulting mixture is allowed to warm to to 25°C and dilute hydrochloric acid ($1.0cm^3$, 2M) added. The aqueous phase is separated, extracted with diethyl ether (2 x $3.0cm^3$), and the extract combined with the organic phase from the reaction. After drying over anhydrous magnesium sulphate and filtering, the solvents are removed by evaporation under reduced pressure the residual oil is purified by layer chromatography.

Method C: Reduction by the method of Yamamoto et al. [ J.Amer. Chem. Soc., 110, 3588, (1988)].

(a) Preparation of the catalyst solution. A solution of trimethylaluminium ($4.2cm^3$, 2.88g, 25% w/w) is added at 0°C to a solution of diterbutylmethylphenol (4.4g) in dry toluene ($10.0cm^3$).

(b) Reduction of the dione. The catalyst solution ($1.5cm^3$) and t-butylmagnesium bromide ($2.2cm^3$ of a 0.5M solution in diethyl ether) is added to a solution of the dione (1 mmole) in dry tetrahydrofuran. When reduction has occurred (the course of the reaction may be monitored by TLC), dilute hydrochloric acid ($2.0cm^3$, 2M) is added, and the product is thereafter obtained by the same procedure as method B. The results are given in the Table below as the ratio of the endo and exo isomeric forms of this hydroxyketone.

| Dione | % ratio of endo: exo isomers in product | | |
|---|---|---|---|
| | Method A | Method B | Method C |
| 3,3,6,6-tetramethylbicyclo [3,1,0]hexa-2,4-dione | 80:20 | 0:100 | 73:27 |
| 3,3-tetramethylbicyclo[3,1,0]hexa-2,4-dione | 92:8 | 55:45 | - |

## Claims

1. A process for preparing a cis-cyclopropane carboxylic acid of formula:

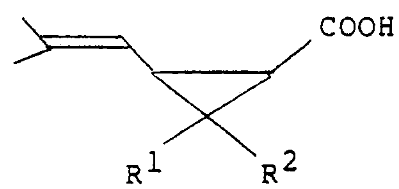

which comprises the step of treating a compound of formula (I):

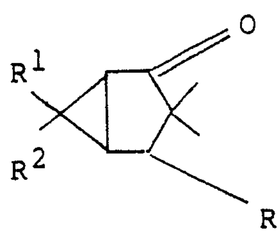

with a source of hydroxide ions in an aqueous medium where R is an atom or group capable of forming a stable anion $R^-$ under the reaction conditions, and $R^1$ and $R^2$ are each selected from hydrogen and methyl.

2. A process according to claim 1 wherein the source of hydroxide ions is an alkali metal hydroxide.

3. A process according to claim 1 wherein the aqueous medium comprises an aprotic solvent.

4. A process according to claim 1 wherein R is an esterified hydroxy group.

5. A process according to claim 4 wherein R is a substituted sulphonyloxy group.

6. A process according to claim 5 wherein R is the methane sulphonyloxy group.

7. A process according to claim 4 wherein the compound of formula I is obtained by esterification of the compound of formula II

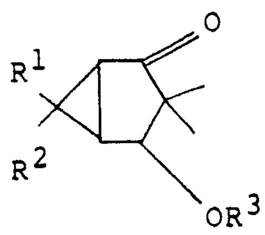

(II)

wherein $R^1$ and $R^2$ are as defined in claim 1 and $R^3$ is hydrogen.

8. A process according to claim 7 wherein the compound of formula II is obtained by treating the compound of formula III

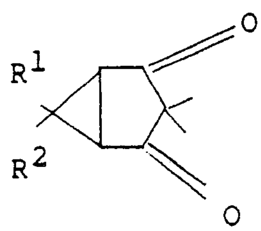

(III)

with a reducing agent.

9. A process according to claim 8 wherein the reducing agent comprises an alkali metal borohydride.

10. A process according to claim 9 carried out in the presence of a cerium salt.

11. A compound of formula II:

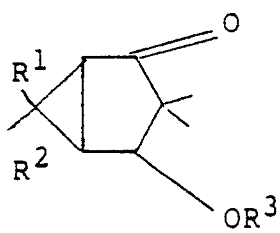

(II)

wherein $R^1$ and $R^2$ are each selected from hydrogen and methyl and $R^3$ is hydrogen or a sulphonyl group.

12. A compound of formula II selected from 4-hydroxy-3,3,6,6-tetramethylbicyclo[3,1,0]hexan-2-one,
4-hydroxy-3,3,6-trimethylbicyclo[3,1,0]hexan-2-one,
4-hydroxy-3,3-dimethylbicyclo[3,1,0]hexan-2-one,
3,3,6,6-tetramethylbicyclo[3,1,0]hexan,-2-on-4-yl methane sulphonate,
3,3,6,6-tetramethylbicyclo[3,1,0]hexan-2-on-4-yl, 4-methylbenzene sulphonate,
3,3,6-trimethylbicyclo[3,1,0]hexan-2-on-4-yl methane sulphonate, and
3,3-dimethylbicyclo[3,1,0]hexan-2-on-4-yl methane sulphonate.

13. An improved process for the preparation of a compound of formula III which comprises the steps of (a) treating a compound of formula IV

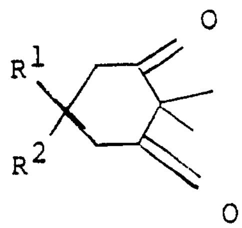

(IV)

wherein $R^1$ and $R^2$ are each selected from hydrogen and methyl, with at least one equivalent of a base in

an aprotic medium, (b) thereafter treating the mixture with bromine, and (c) recovering the compound of formula III from the mixture.

14. 2,2,5-trimethylcyclohex-1,3-dione.

15. 3,3,6-trimethylbicyclo[3,1,0]hexa-2,4-dione.

16. cis-2-methyl-3-(2-methylpropen-1-yl) cyclopropane carboxylic acid.

17. cis-2-(2-methylpropen-1-yl)cyclopropane carboxylic acid.